**Europäisches Patentamt**

**European Patent Office**

**Office européen des brevets**

(11) Numéro de publication: **0 003 446**
**B1**

(12) **FASCICULE DE BREVET EUROPEEN**

(21) Numéro de dépôt: **79400005.9**

(22) Date de dépôt: **04.01.79**

(51) Int. Cl.³: **C 07 D 333/20, C 25 B 3/04**

(54) **Procédé de préparation d'amino-2 éthyl-2 thiophène par voie électrochimique**

(30) Priorité: **25.01.78 FR 7801992**

(43) Date de publication de la demande:
**08.08.79 Bulletin 79/16**

(45) Mention de la délivrance du brevet:
**26.11.80 Bulletin 80/24**

(84) Etats contractants désignés:
**DE NL SE**

(56) Documents cités:
**FR - A - 2 299 332**

(73) Titulaire: **OMNIUM FINANCIER AQUITAINE POUR L'HYGIENE ET LA SANTE SANOFI**
**Tour Aquitaine Cedex No 4**
**F - 92080 Paris La Defense (FR)**

(72) Inventeur: **Braye, Emile Henri Urbain**
**"Puntis"**
**F - 1190 Lagardelle**
**Leze (FR)**

(74) Mandataire: **Lavoix, Jean, et al**
**c/o Cabinet Lavoix 2**
**Place D'Estienne D'Orves**
**F - 75441 Paris Cedex 09 (FR)**

Courier Press, Leamington Spa, England.

## Procéde de préparation d'amino-2 éthyl-2 thiophène par voie électrochimique

La présente invention est relative à un procédé de préparation électrochimique de l'amino-2 éthyl-2 thiophène.

L'amino-2 éthyl-2 thiophène est un produit intermédiaire utilisé dans la synthèse de dérivés de thiénopyridine qui ont notamment des activités inhibitrices de l'agrégation plaquettaire, anti-inflammatoire et vasodilatatrice et sont illustrés dans la brevet français 2 215 948 et son premier certificat d'addition 2 345 150.

Il est déjà connu, d'après la demande française 2 299 332, un procédé de préparation d'amino-2 éthyl-2 thiophène qui consiste à réaliser une réduction à l'aide d'hydrure d'aluminium lithium, mais il ne s'agit pas d'une réaction par voie électrochimique.

Elle a ainsi pour objet un procédé de préparation de l'amino-2 éthyl-2 thiophène, caractérise en ce qu'on réduit par voie électrochimique du nitro-2 vinyl-2 thiophène, dans un milieu organique ou hydro-organique, en présence d'un sel support, à un pH acide et à une température comprise entre 5 et 40°C et on récupère le produit désiré à partir du milieu réactionnel. La récupération du produit est effectuée avantageusement au moyen d'un solvant organique après neutralisation de l'électrolyte.

Le milieu organique doit être tel qu'il permette de solubiliser le produit de départ, à savoir le nitro-2 vinyl-2 thiophène et peut être par exemple de l'acide acétique, du dioxanne ou du méthanol.

On utilise dans un mode de mise en oeuvre préféré un milieu hydro-organique contenant de 20 à 70% en poids de solvant organique et, de préférence, un mélange à 50% de solvant organique, tel qu'un mélange eau-acide acétique 50/50.

La réduction électrochimique est effectuée à un potentiel de 1,10 à 1,30 V mesuré par rapport à une électrode de référence au calomel saturé, et de préférence à un potentiel de 1,15 V environ.

Le pH du milieu réactionnel doit être acide et, de préférence, voisin de 2. Ce pH peut être obtenu par addition d'un acide minéral fort tel que l'acide chlorhydrique ou au moyen d'un tampon approprié (citrate-HCl par exemple).

Le sel support ajouté au milieu organique ou hydro-organique pour réaliser la réaction électrochimique est notamment un chlorure de métal alcalin tel que le chlorure de sodium ou de lithium, à une concentration appropriée qui peut être en particulier de 0,2 à 0,7 mole/l et, de préférence, de 0,5 mole/l.

Une température convenable du milieu réactionnel se situe entre 5 et 40°C et, de préférence, on opère à la température ambiante.

La concentration du nitro-2 vinyl-2 thiophène dans le milieu réactionnel est avantageusement comprise entre 1 g et 10 g/l et, de préférence, entre 1 g et 3 g/l.

Comme électrode de travail pour la cellule électrochimique, on utilise un matériau à forte surtension d'hydrogène tel que le mercure, le zinc ou le plomb, une nappe de mercure étant préférable.

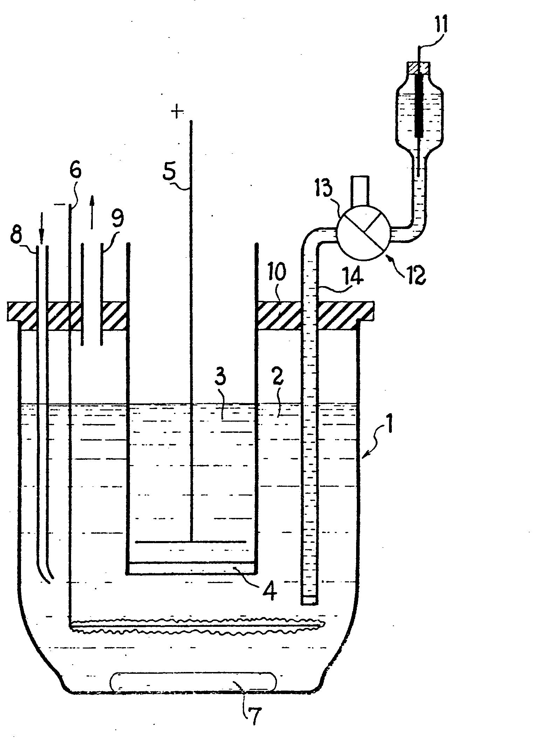

D'autres caractéristiques de l'invention apparaîtront au cours de la description qui va suivre, faite en référence à la figure unique de dessin annexé, donné uniquement à titre d'exemple.

La réduction électrochimique du nitro-2 vinyl-2 thiophène peut être réalisée dans une cellule telle que celle représentée au dessin.

Cette cellule 1 est divisé en un compartiment d'électrolyse 2 et un compartiment anodique 3 qui sont séparés par une paroi 4 poreuse, par exemple en verre fritté, assurant la conduction électrique. Une électrode 5 en platine ou en graphite sert d'anode, alors que la cathode 6 ou électrode de travail est disposée au fond ou à proximité du fond du compartiment d'électrolyse. Le cellule 1 est munie dans son compartiment 2 d'un dispositif d'agitation du milieu réactionnel qui est dans le mode de réalisation représenté, un barreau aimanté 7 disposé au voisinage du fond de la cellule.

La cellule est également munie d'un conduit d'arrivée 8 de gaz inerte débouchant au-dessous du niveau de l'électrolyte et d'une sortie 9 de ce gaz dans le couvercle 10 de la cellule.

Une électrode de référence 11 est placée à l'extérieur de la cellule pour éviter la contamination par les produits de réduction, la liaison électrique étant assurée par un pont 12 comprenant un robinet à trois voies 13 et un tube 14 fermé par un verre fritté plongeant dans le compartiment cathodique 2.

L'exemple non limitatif suivant est donné à titre d'illustration de l'invention.

### EXEMPLE

On réalise la réduction électrochimique du nitro-2 vinyl-2 thiophène dans une cellule à anode de platine, dont l'électrode de travail ou cathode est une nappe de mercure. Le milieu de réaction est un mélange acide acétique-eau à 50% et on utilise comme sel support du chlorure de lithium 0,5 molaire.

Le potentiel de réduction est maintenu à 1,150 V par rapport à une électrode au calomel saturé et la réaction est réalisée à température ambiante.

Le nitro-2 vinyl-2 thiophène est introduit dans le milieu hydro-organique à la concentration initiale de 2,5 g/l.

La cellule est maintenue sous atmosphère d'azote et on effectue alors la réduction, sous agitation, par passage du courant à la tension fixée.

A la fin de l'electrolyse (après passage de 2500 coulombs), on neutralise l'électrolyte du compartiment cathodique, on extrait avec du chloroforme, puis on lave à l'eau et ou sèche sur $K_2CO_3$ anhydre. On récupère l'amine désirée sous forme de chlorhydrate ou d'oxalate dans l'éthanol.

Le rendement en amino-2 éthyl-2 thiophène est de 47% par rapport au produit de départ. Ce rendement correspond également au rendement faradique étant donné que la quantité d'électricité que l'on a fait passer est celle qui correspond à une transformation de 100%.

## Revendications

1. Procédé de préparation d'amino-2 éthyl-2 thiophène, caractérisé en ce qu'on réduit par voie électrochimique, à un potentiel de 1,10 à 1,30 V, mesuré par rapport à une électrode au calomel saturé, du nitro-2 vinyl-2 thiophène, dans un milieu organique ou hydro-organique, en présence d'un sel support qui est un chlorure de métal alcalin présent en une concentration de 0,2 à 0,7 mole/l, à pH acide et à une température comprise entre 5 et 40°C et on récupère le produit désiré à partir du milieu réactionnel.

2. Procédé suivant la revendication 1, caractérisé en ce qu'on récupère l'amino-2 éthyl-2 thiophène du milieu réactionnel au moyen d'un solvant organique, après neutralisation de l'électrolyte.

3. Procédé selon la revendication 1 ou 2, caractérisé en ce que le milieu organique ou hydro-organique est choisi parmi l'acide acétique, le méthanol, le dioxanne ou un mélange de ces derniers avec l'eau en une proportion de 20 à 70% en poids.

4. Procédé suivant la revendication 3, caractérisé en ce qu'on utilise comme milieu un mélange eau-acide acétique 50/50.

5. Procédé selon la revendication 1, caractérisé en ce que la réduction est effectuée à un potentiel de 1,15 V environ.

6. Procédé selon la revendication 1, caractérisé en ce que la concentration du sel support est de 0,5 mole/l.

7. Procédé selon l'une quelconque des revendications précédentes, caractérisé en ce qu'on utilise comme électrodes une cathode en mercure, zinc ou plomb et une anode en platine ou graphite.

8. Procédé selon l'une quelconque des revendications 2 à 7, caractérisé en ce que le solvant d'extraction est du chloroforme.

9. Procédé selon la revendication 1, caractérisé en ce que la concentration initiale en nitro-2 vinyl-2 thiophène est comprise entre 1 et 10 g par litre.

## Patentansprüche

1. Verfahren zur Herstellung von 2-Amino-2-äthyl-thiophen, dadurch gekennzeichnet, daß man ein 2-Nitro-2-vinylthiophen in organischem oder wässrig organischem Medium in Gegenwart eines Alkalimetallchlorids als Salzzusatz in einer Konzentration von 0,2 bis 0,7 mol/l bei saurem pH-Wert und bei einer Temperatur zwischen 5 und 40°C elektrochemisch einer Spannung von 1,10 bis 1,30 V reduziert, und daß man das gewünschte Produkt aus dem Reaktionsmedium isoliert.

2. Verfahren nach Anspruch 1, dadurch gekennzeichnet, daß man das 2-Amino-2-äthyl-thiophen aus dem Reaktionsmedium nach Neutralisieren des Elektrolyten mit einem organischen Lösungsmittel abtrennt.

3. Verfahren nach Anspruch 1 oder 2, dadurch gekennzeichnet, daß man als organisches oder wässrig-oranisches Medium Essigsäure, Methanol, Dioxan oder deren Mischung mit Wasser im Verhältnis von 20 bis 70 Gewichtsprozent verwendet.

4. Verfahren nach Anspruch 3, dadurch gekennzeichnet, daß man als Medium eine Mischung aus Wasser und Essigsäure im Verhältnis 50/50 verwendet.

5. Verfahren nach Anspruch 1, dadurch gekennzeichnet, daß man die Reduktion bei einer Spannung von etwa 1,15 V durchführt.

6. Verfahren nach Anspruch 1, dadurch gekennzeichnet, daß man bei einer Konzentration des Salzzusatzes von 0,5 mol/l arbeitet.

7. Verfahren nach einem der vorhergehenden Ansprüche, dadurch gekennzeichnet, daß man als Elektroden eine Quecksilber-, Zink- oder Bleikathode und eine Platin- oder Graphitanode verwendet.

8. Verfahren nach einem der vorhergehenden Ansprüche 2 bis 7, dadurch gekennzeichnet, daß man Chloroform als Extraktionsmittel verwendet.

9. Verfahren nach Anspruch 1, dadurch gekennzeichnet, daß man von einer Anfangskonzentration von 2-Nitro-2-vinylthiophen von 1 bis 10 g/l ausgeht.

## Claims

1. Process for the preparation of 2-amino-2-ethyl-thiophene, comprising electrochemically reducing 2-nitro-2-vinyl-thiophene at a potential of 1.10—1.30 V, as measured with respect to a calomel saturated reference electrode, within an organic or aqueous-organic medium, in the presence of an electrolyte which is an alkali metal chloride present at a concentration of 0.2—0.7 mole/l, at an acidic pH and at a temperature between 5°C and 40°C, and recovering the desired product from the reaction medium.

2. Process as claimed in claim 1, wherein the 2-amino-2-ethyl-thiophene is recovered from the reaction medium by means of an organic solvent, after neutralization of the electrolyte.

3. Process as claimed in claim 1 or 2, wherein the organic or aqueous-organic medium is selected from acetic acid, methanol, dioxan or mixtures thereof with water in an amount of 20—70 wt%.

4. Process as claimed in claim 3, wherein a water-acetic acid (50:50) mixture is used as medium.

5. Process as claimed in claim 1, wherein the reduction is effected at a potential of about 1.15 V.

6. Process as claimed in claim 1, wherein the electrolyte concentration is of 0.5 mole/l.

7. Process as claimed in any one of the preceding claims, wherein a mercury, zinc or lead cathode and a platinum or graphite anode are used as electrodes.

8. Process as claimed in any one of the preceding claims, wherein the extraction solvent is chloroform.

9. Process as claimed in claim 1, wherein the initial concentration of 2-nitro-2-vinyl-thiophene is 1—10 g/litre.